# EUROPÄISCHE PATENTSCHRIFT

(11) **EP 2 353 625 B1**
(45) Veröffentlichungstag und Bekanntmachung des Hinweises auf die Patenterteilung: **02.07.2014**
(21) Anmeldenummer: 10191337.4
(22) Anmeldetag: 16.11.2010
(51) Int. Cl.: A61M 1/00

(54) **Vorrichtung zum Trennen von Gewebezellen aus einer Flüssigkeit**
Device for separating tissue cells from a liquid
Dispositif de séparation de cellules tissulaires à partir d'un liquide

(30) Priorität: 27.01.2010 DE 102010001292; 15.02.2010 US 304503 P; 17.06.2010 US 817428
(43) Veröffentlichungstag der Anmeldung: 10.08.2011
(73) Patentinhaber: Human Med AG, 19061 Schwerin (DE)
(72) Erfinder: Kensy, Arnd, 14552 Michendorf - OT Wilhelmshorst (DE)
(74) Vertreter: Gulde & Partner

(56) Entgegenhaltungen:
- EP-A1- 0 384 585
- EP-A1- 1 837 045
- EP-A2- 2 119 461
- US-A- 3 685 517

## Beschreibung

Die Erfindung betrifft eine Vorrichtung zum Trennen von Gewebezellen aus einer Flüssigkeit mit einem einen Sammelraum ausbildenden Sammelbehälter, der einerseits mit einer Unterdruckquelle verbunden ist und der andererseits mit einer Zuführleitung für ein Flüssigkeits-Gewebezellen-Gemisch verbunden ist.

Vorrichtungen der gattungsgemäßen Art sind bekannt. So zeigt beispielsweise WO 2009/149691 A2 ein Verfahren und eine Vorrichtung zum Trennen von Gewebezellen aus einer Flüssigkeit, wobei ein unter Vakuum stehender Gewebezellensammler eine Filtereinheit umfasst, die den Sammelbehälter in einem unteren Sammelraum für die Flüssigkeiten und einen mittleren Sammelraum für die Gewebezellen und einen oberen Vakuumraum aufteilt. Der untere Sammelraum für die Flüssigkeit und der obere Vakuumraum sind am Sammelraum für die Gewebezellen vorbei miteinander verbunden.

Der Erfindung liegt die Aufgabe zugrunde, eine Vorrichtung der gattungsgemäßen Art zu schaffen, die sich durch einen einfachen Aufbau auszeichnet und eine schonende Trennung der Gewebezellen aus einem Flüssigkeits-Gewebezellen-Gemisch gestattet.

Erfindungsgemäß wird diese Aufgabe mittels einer Vorrichtung mit den in Anspruch 1 genannten Merkmalen gelöst. Dadurch, dass innerhalb des Sammelraumes eine kommunizierende Röhre angeordnet ist, die in einem Abstand zum Boden des Sammelbehälters mündet, wobei zwischen der kommunizierenden Röhre und dem Sammelraum oberhalb eines sich einstellenden Flüssigkeits-Gewebezellen-Gemischpegels eine Verbindung besteht und innerhalb der kommunizierenden Röhre ein mit der Unterdruckquelle verbundenes Saugelement angeordnet ist, wobei eine Mündung des Saugelementes unterhalb der Verbindung liegt, ist vorteilhaft möglich, in besonders einfacher und schonender Weise eine Trennung der entnommenen Gewebezellen von der Flüssigkeit zu erreichen. Diese schonende Trennung ist insbesondere wichtig, damit die zuvor, beispielsweise einem menschlichen Körper, entnommenen Gewebezellen wieder aufgearbeitet und beispielsweise zum Wiedereinbringen in den menschlichen Körper geeignet sind. Mittels der in den Sammelraum eingreifenden, kommunizierenden Röhre, insbesondere durch das innerhalb der kommunizierenden Röhre angeordnete Saugelement, kann durch den dort anliegenden Unterdruck die Flüssigkeit abgesaugt werden, während sich die Gewebezellen im Sammelraum ablagern. Die Gewebezellen besitzen eine geringere Dichte als die Flüssigkeit, so dass diese durch Auftriebskräfte an die Oberfläche der Flüssigkeit drängen. Da die kommunizierende Röhre möglichst bodennah in den Sammelbehälter eingreift, stellt sich in der kommunizierenden Röhre ein auf gleichem Niveau befindlicher Flüssigkeitsspiegel wie in dem Sammelraum ein. Die Zellen verbleiben hierbei im Sammelraum, während die Flüssigkeit in der kommunizierenden Röhre aufsteigt. Von dort wird diese durch das dort eingreifende Saugelement abgesaugt. Durch die nachfolgende Zufuhr des Flüssigkeits-Gewebezellen-Gemisches ergibt sich über die kommunizierende Röhre dort ein gleicher Flüssigkeitsstand wie in dem Sammelraum. Mittels der Verbindung zwischen der kommunizierenden Röhre und dem Sammelraum oberhalb eines Flüssigkeits-Gewebezellen-Gemischpegels wird dafür Sorge getragen, dass sich in der kommunizierenden Röhre und dem Sammelraum ein Druckgleichgewicht einstellt, so dass hier der Effekt der kommunizierenden Röhre für das Absaugen der Flüssigkeit und das Verbleiben der Gewebezellen im Sammelraum ausgenutzt werden kann. Hierdurch wird die Zuführung des Flüssigkeits-Gewebezellen-Gemisches in den Sammelraum gewährleistet und andererseits die Trennung der Flüssigkeit von den Gewebezellen schonend ermöglicht.

In bevorzugter Ausgestaltung der Erfindung ist vorgesehen, einen möglichst hohen und schlanken Sammelbehälter mit möglichst kleinem Durchmesser für das Auffangen eines Flüssigkeits-Gewebezellen-Gemisches zu verwenden. Hierdurch wird der Auftrieb der Gewebezellen im Flüssigkeitsgemisch konstruktiv begünstigt.

Die im Sammelraum angeordnete kommunizierende Röhre hat oberhalb der Saugöffnung des eingeschlossenen Saugelementes eine Öffnung in den Sammelraum. Diese Öffnung ist verantwortlich für einen schnellen Druckausgleich zwischen der kommunizierenden Röhre mit dem eingeschlossenen Saugelement und dem Sammelraum. Je größer die Öffnung umso geringer ist der Sog, der in der kommunizierenden Röhre auf den Flüssigkeitspegel wirkt. Das beeinflusst den Niveauausgleich in positiver Weise und gibt dadurch dem Flüssigkeits-Gewebezellen-Gemisch im Sammelraum mehr Zeit zur Zelltrennung mittels Auftrieb.

Die im Sammelraum angeordnete kommunizierende Röhre hat des Weiteren einen möglichst kleinen Abstand zum Behälterboden. Über diesen Spalt erfolgt der Niveauausgleich zwischen dem Flüssigkeits-Gewebezellen-Gemisch im Sammelraum und der aufsteigenden Flüssigkeit in der kommunizierenden Röhre. Die Gestaltung des Spaltes ist mitverantwortlich für die korrekte Trennung der Gewebezellen vom Rest des Flüssigkeitsgemisches. Ein kleiner Spalt hat eine Drosselfunktion, wodurch der Niveauausgleich positiv beeinflusst wird, was bedeutet, dass dem Flüssigkeits-Gewebezellen-Gemisch im Sammelraum mehr Zeit zur Zelltrennung mittels Auftrieb gegeben wird.

Beim Beginn der Absaugung des Flüssigkeits-Gewebe-Zellengemisches, das heißt, im Behälter befindet sich noch kein derartiges Gemisch, stellen sich die gewünschten Druckverhältnisse innerhalb des Sammelbehälters schnell ein. Durch den konstruktiven Aufbau des Sammelbehälters bildet sich sofort ein weitestgehend gewebefreier Flüssigkeitsbereich, der über den Spalt zwischen dem Behälterboden und der kommunizierende Röhre zu einem Niveauausgleich zwischen dem Flüssigkeits-Gewebe-Zellengemisch im Sammelraum und dem Innenraum der kommunizierende Röhre führt. Mit fortdauernder Absaugung und steigendem Flüssigkeits-Gewebezellen-Gemisch-Pegel im Sammelraum und in der kommunizierenden Röhre taucht das von der kommunizierenden Röhre umschlossene Saugelement in die abzusaugende Flüssigkeit ein. Um dem Flüssigkeits-Gewebezellen-Gemisch zusätzlich Zeit zur Zelltrennung mittels Auftrieb zu gegeben, erfolgt erst jetzt durch die Öffnung des Saugelementes das Absaugen der Flüssigkeit, die über die Saugleitung in den Flüssigkeitsbehälter befördert wird.

Zum Ende der Absaugung, bei gewünschter Gewebezellmenge im Sammelraum, lässt sich in Abhängigkeit von der eingestellten Eintauchtiefe des Saugelementes der gewebefreie Flüssigkeitsbereich über dem Behälterboden und in der kommunizierenden Röhre verringern, bis sich idealerweise nur noch Gewebezellen im Sammelbehälter befinden.

In weiterer bevorzugter Ausgestaltung der Erfindung ist vorgesehen, dass die kommunizierende Röhre an ihrem dem Boden zugewandten Ende randoffene Ausnehmungen aufweist. Hier wird vorteilhaft möglich, die kommunizierende Röhre sich mit ihrem unteren Ende am Boden des Sammelgefäßes abstützen zu lassen, während die randoffenen Ausnehmungen die Verbindungen zum Übertritt der Flüssigkeit von dem Sammelraum in die kommunizierende Röhre gestattet. Neben einer Lagerung der Röhre im Deckel ist durch diese Abstützung am Boden eine besonders stabile, auf robuste Betriebsweisen angepasste, Vorrichtung geschaffen.

Ferner ist in bevorzugter Ausgestaltung der Erfindung vorgesehen, dass das Saugelement eine variable Länge aufweist und/oder in variabler Länge einstellbar ist. Dadurch wird vorteilhaft möglich, die Vorrichtung auf unterschiedliche zu sammelnde Gewebezellenmengen abzustimmen. Je kürzer das Saugelement ist, umso später gelangt dieses in Kontakt mit dem Flüssigkeitspegel in der kommunizierenden Röhre. Das bedeutet, es tritt zunächst erst eine entsprechend größere Menge des Flüssigkeits-Gewebezellen-Gemisches in den Sammelraum ein, bevor das Absaugen der Flüssigkeit einsetzt. Die Gewebezellen haben hierdurch auch mehr Zeit, aufgrund der Auftriebswirkung sich an der Oberfläche der Flüssigkeit zu sammeln, so dass die Flüssigkeit dann anschließend von unten über die kommunizierende Röhre abgesaugt wird. Auch lassen sich hierdurch größere Gewebezellmengen auffangen als mit einem längeren Saugelement.

Darüber hinaus ist in weiterer bevorzugter Ausgestaltung der Erfindung vorgesehen, dass der Sammelbehälter eine verschließbare Entnahmeöffnung für die gesammelten Gewebezellen umfasst. Hierdurch wird vorteilhaft möglich, die in dem Sammelraum gesammelten Gewebezellen problemlos und schonend zu entnehmen, beispielsweise abzusaugen. Hierdurch muss die gesamte Vorrichtung nicht von der Unterdruckquelle und der Einrichtung zum Zuführen des Flüssigkeits-Gewebezellen-Gemisches getrennt werden. Der Absaugvorgang des Flüssigkeits-Gewebezellen-Gemisches kann für die Entnahme der angesammelten Gewebezellen kurzfristig unterbrochen werden und kann anschließend ohne Neuinstallation fortgesetzt werden. Hierdurch ergibt sich eine besonders effektive Einsatzmöglichkeit für die erfindungsgemäße Vorrichtung.

Schließlich ist in weiterer bevorzugter Ausgestaltung der Erfindung vorgesehen, dass der Sammelbehälter mittels eines Deckels verschließbar ist, der eine Öffnung für den Anschluss der Unterdruckquelle mittels des verstellbaren Saugelementes, eine Öffnung für einen Anschluss der Zuführleitung des Flüssigkeits-Gewebezellen-Gemisches und die verschließbare Entnahmeöffnung aufweist. Hierdurch wird vorteilhaft erreicht, dass alle funktionalen Anschlusselemente in dem Deckel integriert sind und der Sammelbehälter insgesamt sich durch einen einfachen, auch kostengünstig in großer Stückzahl herstellbaren Aufbau auszeichnet.

Ferner ist in bevorzugter Ausgestaltung der Erfindung vorgesehen, dass der Deckel an seiner dem Sammelraum zugewandten Seite Befestigungsmittel für einen Auffangkorb umfasst, der unterhalb der Öffnung für den Anschluss der Zuführleitung für das Flüssigkeits-Gewebezellen-Gemisch angeordnet ist. Hierdurch wird vorteilhaft ermöglicht, dass der Auffangkorb für ein schonendes Überführen des Flüssigkeits-Gewebezellen-Gemisches in den Sammelraum sorgt. Dieses tritt somit nicht mit großer Geschwindigkeit auf die bereits gesammelten Gewebezellen, sondern wird durch den Auffangkorb zunächst im Sinne eines Strömungswiderstandes beruhigt und fließt dann kontrolliert in den Sammelraum ab. Unerwünschte Gewebeanteile, wie z. B. Bindegewebe, werden über den Auffangkorb herausgefiltert und gelangen somit nicht in den Sammelraum.

Die Materialien der erfindungsgemäßen Vorrichtung, insbesondere der Sammelbehälter, der Deckel, die kommunizierende Röhre, das Saugelement und der Auffangkorb sowie die erforderlichen Anschluss- und Verbindungsstücke bestehen aus einem für klinische Einsatzzwecke geeigneten Material, beispielsweise Kunststoff und/oder Metall.

Weitere bevorzugte Ausgestaltungen der Erfindung ergeben sich aus den übrigen, in den Unteransprüchen genannten Merkmalen.

Die Erfindung wird nachfolgend in einem Ausführungsbeispiel anhand der zugehörigen Zeichnungen näher erläutert. Es zeigen:
- Figur 1: eine schematische Darstellung einer Einrichtung zum Flüssigkeitsstrahltrennen mit integrierter erfindungsgemäßer Vorrichtung zum Trennen von Gewebezellen aus einer Flüssigkeit;
- Figur 2: eine schematische Perspektivansicht der erfindungsgemäßen Vorrichtung zum Trennen von Gewebezellen, sowohl im Zusammenbau als auch in Explosionsdarstellung;
- Figur 3: eine Draufsicht auf einen Deckel der Vorrichtung gemäß Figur 2 und
- Figur 4: eine Druntersicht auf den Deckel gemäß der Vorrichtung gemäß Figur 2.

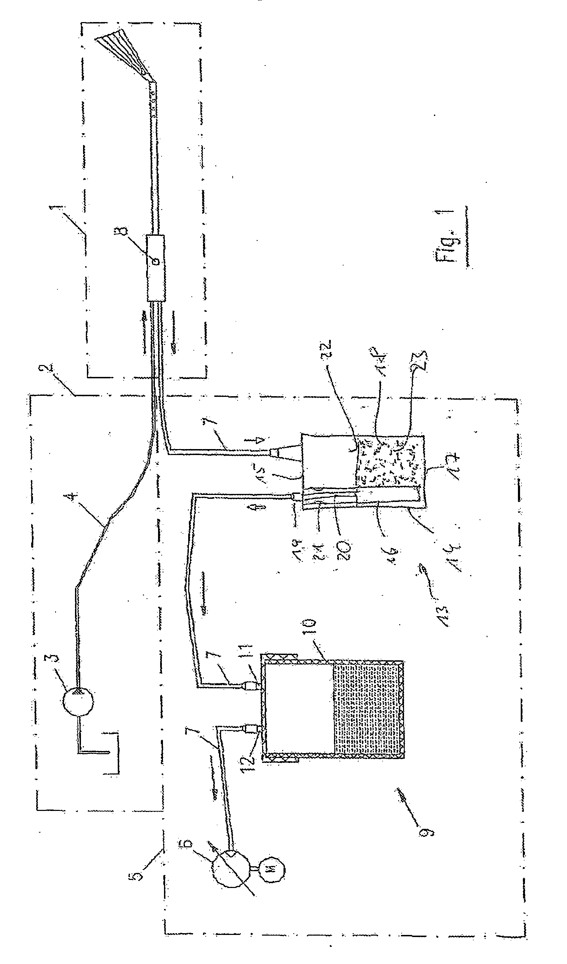

Figur 1 zeigt eine Einrichtung zum Flüssigkeitsstrahltrennen mit einem von der Hand eines Operateurs bedienbaren Applikator 1 zum wasserstrahlunterstützten Trennen und Absaugen von Gewebezellen aus einer biologischen Struktur, mit einer Druckstrahleinrichtung 2, mit einem Druckerzeuger 3 und einer Druckleitung 4 zur Versorgung des Applikators 1 mit einem definierten Flüssigkeitsstrahl sowie einer Saugeinrichtung 5 zur Entsorgung der abgetrennten Gewebeteile (Gewebezellen) und der verwendeten Arbeitsflüssigkeit und der körpereigenen Flüssigkeit aus dem Körper 1. Die Saugeinrichtung 5 umfasst einen Vakuumerzeuger 6 und eine Saugleitung 7, wobei die Saugleitung 7 den Vakuumerzeuger 6 mit dem Applikator 1 durchgehend verbindet. Im Bereich des Applikators 1 besitzt die Saugleitung 7 einen verschließbaren Bypass 8, der die Saugleitung 7 mit der Atmosphäre verbindet. In der Saugleitung 7 befindet sich ein Restflüssigkeitssammler 9 für die abgesaugte Flüssigkeit mit einem verschließbaren Auffangbehälter 10 sowie einem Eingangsanschluss 11 und einem Ausgangsanschluss 12 für die Saugleitung 7. Der Restflüssigkeitssammler 9 für die ausgefilterte Flüssigkeit ist in Saugrichtung vor dem Vakuumerzeuger 6 angeordnet. Zwischen dem Restflüssigkeitssammler 9 und dem Applikator 1 ist ein Gewebezellensammler 13 in die Saugleitung 7 geschaltet.

Der Gewebezellensammler 13 besteht aus einem zylindrischen Sammelbehälter 14, der mit einem Deckel 15 druckdicht verschlossen ist. Der Sammelbehälter 14 ist vorzugsweise zylindrisch ausgebildet. Vorzugsweise ist der Sammelbehälter 14 zur Beobachtung des Füllstandes und zur Bewertung des Zustandes der aufgefangenen Gewebezellen transparent ausgeführt. Zur Beobachtung des Füllstandes besitzt der Sammelbehälter 14 eine Füllstandsanzeige.

Im Sammelbehälter 14 ist eine kommunizierende Röhre 16 angeordnet. Die kommunizierende Röhre 16 mündet in einem Abstand über einem Boden 17 des Sammelbehälters 14. Hierdurch kommt es zur Ausbildung einer (unteren) Verbindung zwischen der kommunizierenden Röhre 16 und einem Sammelraum 18 innerhalb des Sammelbehälters 14. Die kommunizierende Röhre 16 ist an dem Deckel 15 befestigt und umgreift einen Anschluss 19 für die Saugleitung 7 in Richtung des Vakuumerzeugers 6 (Unterdruckquelle). Die kommunizierende Röhre 16 umgreift eine in Figur 1 nicht gezeigte Öffnung für den Anschluss der Saugleitung 7. Diese Öffnung ist unmittelbar mit einem Saugelement 20 verbunden, das sich in die kommunizierende Röhre 16 hinein erstreckt. Die kommunizierende Röhre 16 besitzt wenigstens eine Öffnung 21, so dass es zu einer (oberen) Verbindung zwischen dem Innenraum der kommunizierenden Röhre 16 und dem Sammelraum 18 kommt. Diese wenigstens eine Öffnung 21 befindet sich oberhalb eines hier angedeuteten Pegels 22 eines Flüssigkeits-Gewebezellen-Gemisches 23 innerhalb des Sammelbehälters 14. Die Länge des Saugelementes 20 bestimmt die Höhe des Pegels 22.

Die in Figur 1 dargestellte Einrichtung zum Flüssigkeitsstrahltrennen zeigt folgende Funktion:

Bei Anwendung des Flüssigkeitsstrahltrennverfahrens tritt aus dem Applikator 1 ein definierter Flüssigkeitstrennstrahl aus, dessen Wirkung durch den in der Druckstrahleinrichtung erzeugten Flüssigkeitsdruck und die konstruktive Ausführung des Applikators 1 bestimmt wird. Diese Wirkung ist darauf gerichtet, Gewebezellen aus einer biologischen Struktur in schonender Weise herauszutrennen. Die so abgetrennten Gewebezellen werden zusammen mit der eingespritzten Arbeitsflüssigkeit und den weiteren körpereigenen Flüssigkeiten durch ein in der Saugeinrichtung 5 erzeugtes Vakuum abgesaugt. Dieses Verfahren wird häufig bei der Fettabsaugung angewendet. Immer dann, wenn die derart abgesaugten Gewebezellen einer Wiederverwendung zugeführt werden sollen, werden diese Gewebezellen aus dem Flüssigkeits-Gewebezellen-Gemisch separiert. Dies geschieht durch den Gewebezellensammler 13.

Bei einer Stand-By-Stellung hält der Operateur den verschließbaren Bypass 8 geöffnet, so dass keine Absaugung erfolgt, sondern nur atmosphärische Luft angesaugt und durch den Gewebezellensammler 13 transportiert wird. Hierbei passiert die Luft den Gewebezellensammler 13 über den oberen Bereich des Sammelraumes 18, die kommunizierende Röhre 16 mit der Öffnung 21 sowie das Saugelement 20 in Richtung des Vakuumerzeugers 6.

In einer Betriebsstellung ist der verschließbare Bypass 8 durch den Operateur verschlossen, so dass sich die Saugkraft des Vakuumerzeugers 6 auf das Operationsfeld überträgt. Hierdurch werden die abgetrennten Gewebeteile (Gewebezellen) und die verschiedenen Flüssigkeiten aufgenommen und zum Gewebezellensammler 13 transportiert. Im Gewebezellensammler 13 gelangt dieses Gemisch aus Gewebezellen und Flüssigkeit über den Eingangsstutzen in den Sammelraum 18. Innerhalb des Sammelraums 18 steigt der Pegel des Flüssigkeits-Gewebezellen-Gemisches 23 an, wobei die Gewebezellen aufgrund ihrer geringeren Dichte eine Auftriebskraft erfahren und innerhalb des Flüssigkeits-Gewebezellen-Gemisches 23 an die Oberfläche aufsteigen, also in Richtung des Pegels 22 drängen. In der kommunizierenden Röhre 16 herrscht der gleiche Pegelstand wie im Sammelraum 18. Über die Verbindung zwischen dem unteren Ende der kommunizierenden Röhre in Nähe des Bodens 17 und dem Sammelraum 18 steigt in der kommunizierenden Röhre im Wesentlichen nur die Flüssigkeit des Flüssigkeits-Gewebezellen-Gemisches 23 auf. Die Gewebezellen verbleiben im Sammelraum 18 während die Flüssigkeit in der kommunizierenden Röhre 16 aufsteigt. Nachdem der Pegel 22 so weit angestiegen ist, dass die Mündung des Saugelementes 20 erreicht ist, wird die Flüssigkeit innerhalb der kommunizierenden Röhre 16 in Richtung des Auffangbehälters 10 abgesaugt. Dieser Vorgang wird so weit fortgesetzt, bis im Sammelraum 18 die gewünschte oder die maximal mögliche Menge an Gewebezellen gesammelt wurde. Es wird deutlich, dass die Länge des Saugelementes 20 die Höhe des Pegelstandes 22 beeinflusst. Je kürzer das Saugelement 20, umso höher kann der Pegel 22 innerhalb des Sammelbehälters 14 liegen und umso größer ist die ansammelbare Menge an Gewebezellen innerhalb des Sammelraums 18.

Über die wenigstens eine Öffnung 21 zwischen der kommunizierenden Röhre 16 und dem Sammelraum 18 wird ein Druckausgleich zwischen dem Sammelraum 18 und der kommunizierenden Röhre erreicht, so dass Flüssigkeit in der kommunizierenden Röhre - aufgrund der Gesetzmäßigkeit der kommunizierenden Röhren - zu dem gleichen Pegel aufsteigt wie das Flüssigkeits-Gewebezellen-Gemisch 23 im Sammelraum 18.

Der freie Querschnitt zwischen der kommunizierenden Röhre 16 im Bereich des Bodens 17 und dem Sammelraum 18 ist möglichst so gering bemessen, dass durch diesen nur die Flüssigkeit aber im Wesentlichen keine Gewebezellen in die kommunizierende Röhre 16 gelangen können. Nach einem nicht gezeigten Ausführungsbeispiel kann hierzu die kommunizierende Röhre 16 an ihrem unteren Ende randoffene Aufnehmungen aufweisen, so dass sich die Röhre 16 am Boden 17 abstützen kann und trotzdem ein freier Querschnitt zwischen der kommunizierenden Röhre 16 und dem Sammelraum 18 zum Übertritt der Flüssigkeit verbleibt.

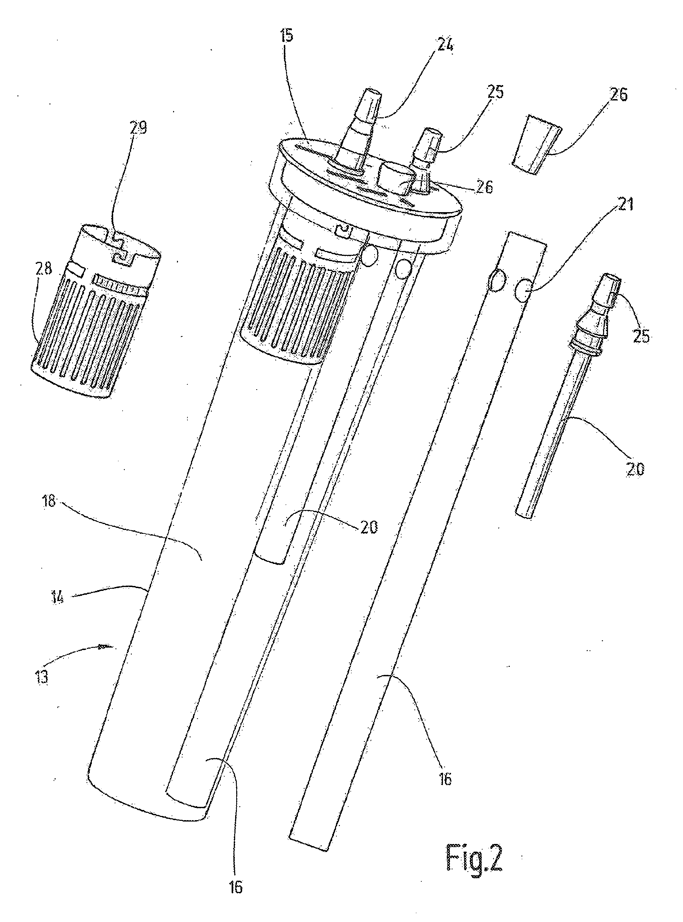

Figur 2 zeigt eine schematische Perspektivansicht des Sammelbehälters 14, einmal in der Zusammenbausituation und in Einzeldarstellung einiger Teile. Gleiche Teile wie in Figur 1 sind mit gleichen Bezugszeichen versehen und nicht nochmals erläutert.

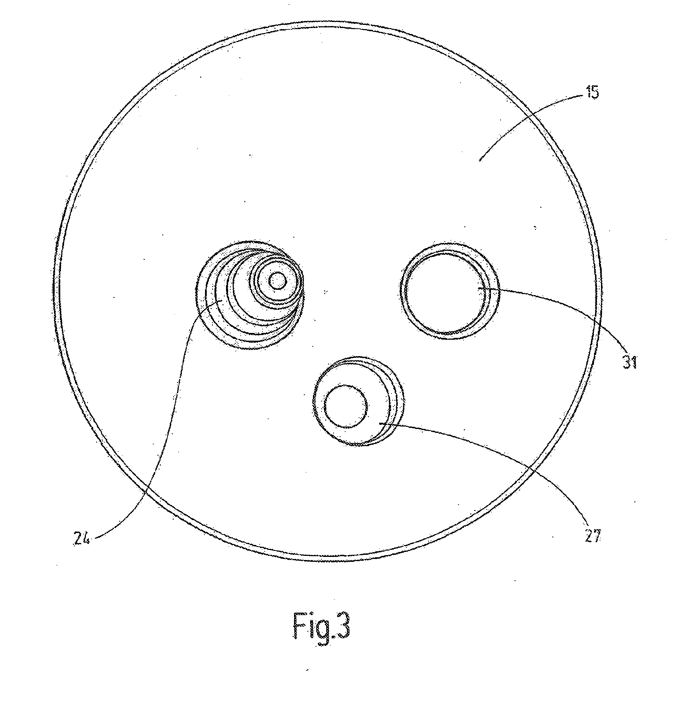

Es wird deutlich, dass der Sammelbehälter 14 durch den Deckel 15 druckdicht verschlossen ist. Der Deckel 15 besitzt ein Anschlusselement 25 zur Verbindung über die Saugleitung 7 mit dem Auffangbehälter 10. Ferner ist ein Anschlusselement 24 dargestellt zur Verbindung der Saugleitung 7 in Richtung des Applikators 1. Das Anschlusselement 25 ist mit dem Saugelement 20 verbunden. Ferner ist ein Verschlussstopfen 26 gezeigt, mittels dem eine Öffnung 27 (Figur 3) im Deckel 15 verschließbar ist. Zu erkennen ist ferner die kommunizierende Röhre 16, innerhalb der das Saugelement 20 angeordnet ist. Die kommunizierende Röhre 16 besitzt die Öffnungen 21 zur (oberen) Verbindung mit dem Sammelraum 18.

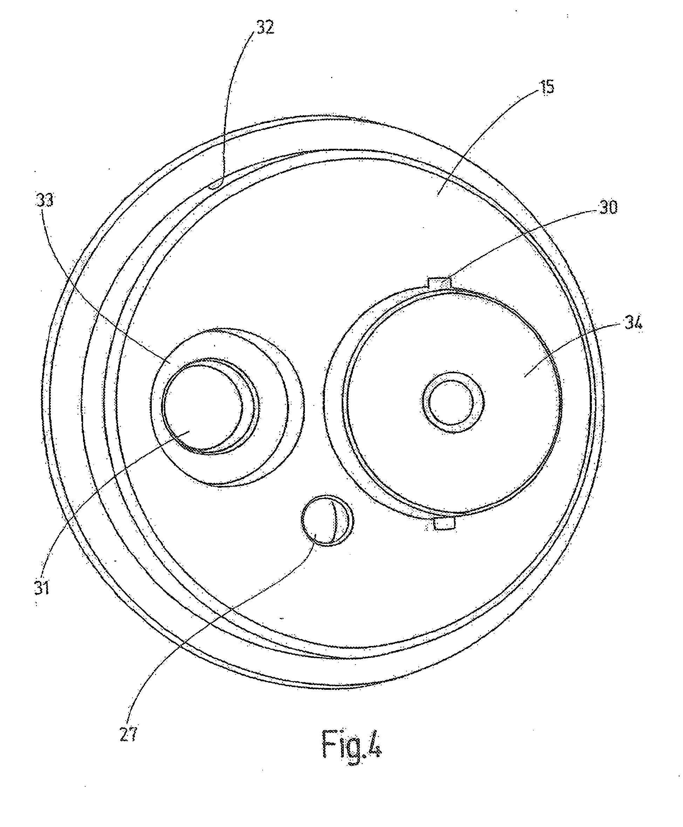

Figur 2 zeigt ferner einen Auffangkorb 28, der an der Unterseite des Deckels 15 befestigbar ist. Hierzu besitzt der Auffangkorb 28 Rastverbindungen 29 nach Art eines Bajonettverschlusses, die mit entsprechenden Zapfen 30 (Figur 4) an der Unterseite des Deckels korrespondieren. Der Auffangkorb 28 besitzt eine im Wesentlichen zylindrische Gestalt mit über dem Umfang angeordneten Durchbrüchen. Der Auffangkorb 28 ist direkt unterhalb des Anschlusses 24 angeordnet. Auf diese Weise gelangt das über den Applikator 1 abgesaugte Flüssigkeits-Gewebezellen-Gemisch 23 zunächst in den Auffangkorb 28 und gelangt von diesem dann in den eigentlichen Sammelraum 18. Der Auffangkorb 28 dient einer Beruhigung des Flüssigkeits-Gewebezellen-Gemisches 23, das über die Saugleitung 7 in dem Sammelbehälter 14 gelangt. Insofern werden die im Sammelraum 18 bereits gesammelten Gewebezellen nicht direkt mit der Strömung des einfließenden Flüssigkeits-Gewebezellen-Gemisches 23 belastet.

In den Figuren 3 und 4 ist der Deckel 15 jeweils in einer Draufsicht und einer Druntersicht gezeigt. Die Draufsicht in Figur 3 verdeutlicht die Öffnung 27, in die der Verschlussstopfen 26 zum druckdichten Verschluss einsetzbar ist. Hierzu sind entsprechend ausgebildete konische Anlageflächen vorgesehen. Bei Entfernen des Verschlussstopfens 26 besteht über die Öffnung 27 ein Zugang zu dem Sammelraum 18. Über eine entsprechende Sauglanze oder dergleichen können dann die im Sammelbehälter 14 angesammelten Gewebezellen entnommen werden beziehungsweise einer weiteren Verarbeitung zugeführt werden.

Ersichtlich ist ferner eine Öffnung 31, in die von der Oberseite des Deckels 15 das Anschlusselement 25 mit dem daran befestigten Saugelement 20 druckdicht eingesetzt wird. Darüber hinaus ist das mit dem Deckel 15 verbundene Anschlusselement 24 erkennbar, das der Verbindung mit der Saugleitung 7 zum Applikator 1 dient.

Die Druntersicht unter den Deckel 15 in Figur 4 verdeutlicht, dass der Deckel 15 eine umlaufende Ringstufe 32 aufweist, die ein druckdichtes Einsetzen in den Sammelbehälter 14 gestattet. Ferner ist erkennbar, dass um die Öffnung 31 eine ringförmige Vertiefung 33 angeordnet ist. In diese kommt die kommunizierende Röhre 16 zur Anlage und wird durch den Deckel 15 fixiert. Gleichzeitig wird das Anschlusselement 25 durch die Öffnung 31 geführt und in dieser druckdicht gehalten. Erkennbar ist ein an der Unterseite des Deckels 15 nach unten überspringender Bereich 34, der diametral gegenüberliegend die Bolzen 30 zur Aufnahme des Auffangkorbes 28 aufweist.

### Bezugszeichenliste

- 1: Applikator
- 2: Druckstrahleinrichtung
- 3: Druckerzeuger
- 4: Druckleitung
- 5: Saugeinrichtung
- 6: Vakuumerzeuger
- 7: Saugleitung
- 8: Bypass
- 9: Restflüssigkeitssammler
- 10: Auffangbehälter
- 11: Eingangsanschluss
- 12: Ausgangsanschluss
- 13: Gewebezellensammler
- 14: Sammelbehälter
- 15: Deckel
- 16: kommunizierende Röhre
- 17: Boden
- 18: Sammelraum
- 19: Anschluss
- 20: Saugelement
- 21: Öffnung
- 22: Pegel
- 23: Flüssigkeits-Gewebezellen-Gemisch
- 24: Anschlusselement
- 25: Anschlusselement
- 26: Verschlussstopfen
- 27: Öffnung
- 28: Auffangkorb
- 29: Rastverbindung
- 30: Zapfen/Bolzen
- 31: Öffnung
- 32: Ringstufe
- 33: ringförmige Vertiefung
- 34: überspringender Bereich

## Patentansprüche

1. Vorrichtung zum Trennen von Gewebezellen aus einer Flüssigkeit, mit einem einen Sammelraum ausbildenden Sammelbehälter, der einerseits mit einer Unterdruckquelle verbunden ist und der andererseits mit einer Zuführleitung für das Flüssigkeits-Gewebezellen-Gemisch verbunden ist,
**dadurch gekennzeichnet, dass**
innerhalb des Sammelraumes (18) eine kommunizierende Röhre (16) angeordnet ist, die in einem Abstand zum Boden (17) des Sammelbehälters (14) mündet, wobei zwischen der kommunizierenden Röhre (16) und dem Sammelraum (18) oberhalb eines sich im Sammelbehälter (14) einstellenden Flüssigkeits-Gewebezellen-Gemischpegels (22) eine Verbindung (21) besteht und innerhalb der kommunizierenden Röhre (16) ein mit der Unterdruckquelle (6) verbundenes Saugelement (20) angeordnet ist, wobei eine Mündung des Saugelementes (20) unterhalb der Verbindung (21) liegt.

2. Vorrichtung nach Anspruch 1,
**dadurch gekennzeichnet, dass**
der Abstand der kommunizierenden Röhre (16) zum Boden (17) des Sammelbehälters (14) so gewählt ist, dass ein freier Querschnitt zwischen der kommunizierenden Röhre (16) und dem Sammelraum (18) am Boden (17) kleiner ist als ein freier Querschnitt der Verbindung (21) zwischen der kommunizierenden Röhre (16) und dem Sammelraum (18) oberhalb des Flüssigkeits-Gewebezellen-Gemischpegels (22).

3. Vorrichtung nach einem der vorhergehenden Ansprüche,
**dadurch gekennzeichnet, dass**
die kommunizierende Röhre (16) an ihrem dem Boden (17) zugewandten Ende randoffene Ausnehmungen aufweist.

4. Vorrichtung nach einem der vorhergehenden Ansprüche,
**dadurch gekennzeichnet, dass**
das Saugelement (20) eine variable Länge aufweist und/oder in variabler Länge einstellbar ist.

5. Vorrichtung nach einem der vorhergehenden Ansprüche,
**dadurch gekennzeichnet, dass**
der Sammelbehälter (14) eine verschließbare Entnahmeöffnung (27) für die gesammelten Gewebezellen umfasst.

6. Vorrichtung nach einem der vorhergehenden Ansprüche,
**dadurch gekennzeichnet, dass**
der Sammelbehälter (14) mittels eines Deckels (15) verschließbar ist, der eine Öffnung (31) für den Anschluss der Unterdruckquelle, ein Anschlusselement (24) zum Anschluss einer Zuführleitung für das Flüssigkeits-Gewebezellen-Gemisch (23) und die verschließbare Entnahmeöffnung (27) aufweist.

7. Vorrichtung nach einem der vorhergehenden Ansprüche,
**dadurch gekennzeichnet, dass**
der Deckel (15) an seiner dem Sammelraum (18) zugewandten Seite Befestigungsmittel (30) für einen Auffangkorb (28) umfasst.

## Claims

1. A device for separating tissue cells from a liquid including a collecting container defining a collection chamber which on the one hand is connected to a negative pressure source and on the other hand is connected to a supply line for the liquid-tissue cell mixture,
**characterized in that**
a communicating tube (16) that ends at a distance from the bottom (17) of the collecting container (14) inside the collection chamber (18), wherein there is a connection (21) between the communicating tube (16) and the collection chamber (18) above a liquid-tissue cell mixture level (22) that is formed in the collecting container (14) and a suction element (20) connected to the negative pressure source (6) is disposed inside the communicating tube (16), wherein a mouth of the suction element (20) is located below the connection (21).

2. The device according to claim 1,
**characterized in that**
the distance of the communicating tube (16) from the bottom (17) of the collecting container (14) is selected such that a free cross section between the communicating tube (16) and the collection chamber (18) at the bottom (17) is smaller than a free cross section of the connection (21) between the communicating tube (16) and the collection chamber (18) above the liquid-tissue cell mixture level (22).

3. The device according to any one of the preceding claims,
**characterized in that**
the communicating tube (16) comprises recesses that are open at the edges on its end facing the bottom (17).

4. The device according to any one of the preceding claims,
**characterized in that**
the suction element (20) has a variable length and/or can be set to variable length.

5. The device according to any one of the preceding claims,
**characterized in that**
the collecting container (14) includes a lockable removal opening (27) for the collected tissue cells.

6. The device according to any one of the preceding claims,
**characterized in that**
the collecting container (14) can be closed using a lid (15) which comprises an opening (31) for connecting the negative pressure source, a connecting element (24) for connecting a supply line for the liquid-tissue cell mixture (23), and the lockable removal opening (27).

7. The device according to any one of the preceding claims,
**characterized in that**
the lid (15) comprises a fastening means (30) for a collection basket (28) on its side facing the collection chamber (18).

## Revendications

1. Procédé permettant d'isoler des cellules tissulaires contenues dans un liquide, avec un réservoir qui forme une chambre collectrice et qui est raccordé d'un côté à une source de pression négative et d'un autre côté à une conduite d'alimentation destinée au mélange liquide-cellules tissulaires,
**caractérisé en ce que**,
à l'intérieur de la chambre collectrice (18), il est disposé un tube (16) communiquant qui débouche à une distance du fond (17) du réservoir (14), un raccordement (21) existant entre le tube (16) communiquant et la chambre collectrice (18) au-dessus d'un niveau de mélange liquide-cellules tissulaires (22) qui s'établit dans le réservoir (14), et un élément d'aspiration (20) raccordé à la source de pression négative (6) étant disposé à l'intérieur du tube (16) communiquant, une embouchure de l'élément d'aspiration (20) étant située au-dessous du raccordement (21).

2. Dispositif selon la revendication 1,
**caractérisé en ce que**
la distance séparant le tube (16) communiquant et le fond (17) du réservoir (14) est choisie de telle sorte qu'une section transversale libre entre le tube (16) communiquant et la chambre collectrice (18) sur le fond (17) est plus petite qu'une section transversale libre du raccordement (21) entre le tube (16) communiquant et la chambre collectrice (18) au-dessus du niveau de mélange liquide-cellules tissulaires (22).

3. Dispositif selon une des revendications précédentes,
**caractérisé en ce que**
à son extrémité tournée vers le fond (17), le tube (16) communiquant présente des creux à bord ouvert.

4. Dispositif selon une des revendications précédentes,
**caractérisé en ce que**
l'élément d'aspiration (20) présente une longueur variable et/ou peut être réglé à une longueur variable.

5. Dispositif selon une des revendications précédentes,
**caractérisé en ce que**
le réservoir (14) comprend une ouverture de prélèvement (27) pouvant être fermée et destinée aux cellules tissulaires collectées.

6. Dispositif selon une des revendications précédentes,
**caractérisé en ce que**
le réservoir (14) peut être fermé au moyen d'un couvercle (15) qui présente une ouverture (31) pour le raccordement de la source de pression négative, un élément de raccordement (24) pour le raccordement d'une conduite d'alimentation pour le niveau de mélange liquide-cellules tissulaires (23), et l'ouverture de prélèvement (27) pouvant être fermée.

7. Dispositif selon une des revendications précédentes,
**caractérisé en ce que**,
sur son côté tourné vers la chambre collectrice (18), le couvercle (15) comprend des moyens de fixation (30) pour un panier de réception (28).
